# EUROPEAN PATENT APPLICATION

(11) **EP 4 809 831 A1**
(43) Date of publication of application: **23.09.2026**
(21) Application number: 24891887.2
(22) Date of filing: 18.11.2024
(51) Int. Cl.: A23L 33/135, A23L 33/105, A23L 33/125, A23L 33/15, A61K 35/747, A61K 35/745, A61K 31/733, A61K 31/525, A61P 25/20, A61P 1/00

(54) **COMPOSITION FOR PREVENTING OR ALLEVIATING SLEEP DISORDERS USING COMBINATION REGIMEN, COMPRISING LACTOBACILLUS SP. STRAIN AND KOREAN MEDICINAL HERBS**

(30) Priority: 17.11.2023 KR 20230159700
(71) Applicant: GI Longevity Inc., Seongnam-si, Gyeonggi-do 13201 (KR); Seoul National University R&DB Foundation, Seoul 08826 (KR)
(72) Inventor: YANG, Bo Gie, Seoul 05849 (KR); HONG, Yun-Chul, Seoul 07322 (KR)
(74) Representative: Vossius & Partner Patentanwälte Rechtsanwälte mbB
(86) International application number: PCT/KR2024/096624
(87) International publication number: WO 2025/105941

(57) **Abstract**

The present disclosure relates to a composition for preventing or alleviating a sleep disorder using a combination therapy including a *Lactobacillus* sp. strain and an herbal medicinal material. According to the composition for preventing or alleviating a sleep disorder, in which a *Lactobacillus* sp. strain and an herbal medicinal material are administered in combination according to one aspect, the composition may be useful for preventing or treating a sleep disorder-related disease, such as alleviating a defecation disorder symptom caused by a sleep disorder or preventing or alleviating depression caused by a sleep disorder.

## Description

### Technical Field

The present disclosure relates to a composition for preventing or alleviating a sleep disorder, in which a *Lactobacillus* sp. strain and an herbal medicinal material are administered in combination.

### Background Art

A sleep disorder is one of the problems commonly experienced by modern people, and is a very common disease that about 20 % or more of the population has experienced or is suffering from. Such a sleep disorder refers to a state of failing to get healthy sleep, a state of failing to maintain alertness during the day despite getting sufficient sleep, or a state of experiencing difficulties when sleeping or being awake due to a disrupted sleep rhythm. A psychological problem is also a major cause, and the sleep disorder is caused by excessive stress, anxiety, tension, fear, and the like.

The sleep disorder may cause various personal and social problems, and is a cause of a learning disability, a decrease in efficiency, a traffic accident, a safety accident, an emotional disorder, a social maladjustment, dissatisfaction with married life, an industrial accident, and the like. In addition, if the sleep disorder is not properly treated, it may cause a defecation disorder such as constipation and irritable bowel syndrome, depression, restless legs syndrome, myocardial infarction, a stroke, and the like.

Meanwhile, the sleep disorder may also occur due to aging, and such a sleep disorder may lead to an additional health problem such as a defecation disorder or depression along with various physiological changes in the body. Generally, as aging progresses, a biological rhythm and a sleep pattern of the body change, and thus sleep quality deteriorates, which increases a possibility of occurrence of various sleep disorders. In addition, if the sleep disorder persists, an autonomic nervous system that regulates an intestinal function is affected, and thus a defecation disorder may appear. Furthermore, a lack of sleep and irregular sleep may cause emotional instability, thereby increasing a risk of developing depression.

To treat such a sleep disorder, a chemical sleeping pill that induces sleep and maintains a sleep state by reversibly suppressing a central nervous system is used. A conventional sleeping pill has a central nervous system inhibitory action like an anesthetic, and thus acts as a sedative in a low dose and exerts a hypnotic action in a moderate amount, but causes coma, paralysis, and respiratory depression in a large amount. A barbiturate-based drug has low safety, and thus tolerance and dependence easily occur, and there is a problem in that if the drug is discontinued after long-term continuous use, a sleep disorder caused by nightmares or the like occurs.

Therefore, for a long-term user of a sleeping pill and a person restricted from using a sleeping pill, a need for a means capable of replacing the sleeping pill is increasing, and a demand for a composition for preventing or alleviating a sleep disorder with fewer side effects is increasing.

### Disclosure of Invention

### Technical Problem

One aspect provides a composition for preventing or alleviating a sleep disorder, containing, as active ingredients, a first active material including a *Lactobacillus* sp. strain or a culture thereof and a second active material including an herbal medicinal material.

Another aspect provides a composition for preventing or alleviating a sleep disorder, containing a first active material including a *Lactobacillus* sp. strain or a culture thereof as an active ingredient, wherein a second active material including an herbal medicinal material is administered in combination.

Another aspect provides a composition for preventing, alleviating, or treating a sleep disorder-related disease, containing, as active ingredients, a first active material including a *Lactobacillus* sp. strain or a culture thereof and a second active material including an herbal medicinal material.

Another aspect provides a composition for preventing, alleviating, or treating a sleep disorder-related disease, containing a first active material including a *Lactobacillus* sp. strain or a culture thereof as an active ingredient, wherein a second active material including an herbal medicinal material is administered in combination.

Another aspect provides a health functional food for preventing or alleviating a defecation disorder or depression, containing, as active ingredients, a first active material including a *Lactobacillus* sp. strain or a culture thereof and a second active material including an herbal medicinal material.

Another aspect provides a health functional food for preventing or alleviating a defecation disorder or depression, containing a first active material including a *Lactobacillus* sp. strain or a culture thereof as an active ingredient, wherein a second active material including an herbal medicinal material is administered in combination.

Another aspect provides a pharmaceutical composition for preventing or treating a defecation disorder or depression, containing, as active ingredients, a first active material including a *Lactobacillus* sp. strain or a culture thereof and a second active material including an herbal medicinal material.

Another aspect provides a pharmaceutical composition for preventing or treating a defecation disorder or depression, containing a first active material including a *Lactobacillus* sp. strain or a culture thereof as an active ingredient, wherein a second active material including an herbal medicinal material is administered in combination.

Another aspect provides a method of preventing or alleviating a sleep disorder, including administering, to a subject in need thereof, a composition containing, as active ingredients, a first active material including a *Lactobacillus* sp. strain or a culture thereof and a second active material including an herbal medicinal material.

Another aspect provides a use of a composition for preventing or alleviating a sleep disorder, the composition containing, as active ingredients, a first active material including a *Lactobacillus* sp. strain or a culture thereof and a second active material including an herbal medicinal material.

Another aspect provides a use of a composition for the manufacture of a medicament for preventing or alleviating a sleep disorder, the composition containing, as active ingredients, a first active material including a *Lactobacillus* sp. strain or a culture thereof and a second active material including an herbal medicinal material.

Another aspect provides a method of preventing or alleviating a sleep disorder, including: administering a first active material including a *Lactobacillus* sp. strain or a culture thereof to a subject in need thereof; and administering a second active material including an herbal medicinal material to the subject in need thereof.

Another aspect provides a use of a composition for preventing or alleviating a sleep disorder, the composition containing a first active material including a *Lactobacillus* sp. strain or a culture thereof as an active ingredient, wherein a second active material including an herbal medicinal material is administered in combination.

Another aspect provides a use of a composition for the manufacture of a medicament for preventing or alleviating a sleep disorder, the composition containing a first active material including a *Lactobacillus* sp. strain or a culture thereof as an active ingredient, wherein a second active material including an herbal medicinal material is administered in combination.

Another aspect provides a use of a composition for preventing or alleviating a sleep disorder, the composition containing, as active ingredients, a first active material including a *Lactobacillus* sp. strain or a culture thereof and a second active material including an herbal medicinal material; or containing the first active material as an active ingredient, wherein the second active material is administered in combination.

Another aspect provides a use of a composition for the manufacture of a medicament for preventing or alleviating a sleep disorder, the composition containing, as active ingredients, a first active material including a *Lactobacillus* sp. strain or a culture thereof and a second active material including an herbal medicinal material; or containing the first active material as an active ingredient, wherein the second active material is administered in combination.

### Solution to Problem

One aspect provides a composition for preventing or alleviating a sleep disorder, or a pharmaceutical composition for preventing, alleviating, or treating a sleep disorder-related disease, containing, as active ingredients, a first active material including a *Lactobacillus* sp. strain or a culture thereof and a second active material including an herbal medicinal material; or containing the first active material as an active ingredient, wherein the second active material is administered in combination.

As used herein, the term *Lactobacillus* refers to a microorganism of an aerobic or facultatively anaerobic Gram-positive bacillus genus widely distributed in nature, and the *Lactobacillus* sp. strain is not limited in kind as long as the strain is a *Lactobacillus* sp. strain known in the art.

The *Lactobacillus* has been renamed to *Limosilactobacillus* or *Lactiplantibacillus*, and the changed strain names may be used interchangeably herein. For example, the strain name of *Lactobacillus fermentum* has been changed to *Limosilactobacillus fermentum*, and the strain name of *Lactobacillus plantarum* has been changed to *Lactiplantibacillus plantarum*.

As used herein, the *Lactobacillus* sp. refers to the genus *Lactobacillus* prior to reclassification, which includes *Lactiplantibacillus plantarum* and *Limosilactobacillus fermentum*.

In an embodiment, the first active material may be Lactobacillus fermentum and *Lactobacillus plantarum*.

In an embodiment, the strain may be a *Lactobacillus fermentum* GB102 strain belonging to the *Lactobacillus* sp. deposited under accession number KCTC 14105BP, a *Lactobacillus fermentum* GB103 strain belonging to the *Lactobacillus* sp. deposited under accession number KCTC 14106BP, or a *Lactobacillus plantarum* GB104 strain belonging to the *Lactobacillus* sp. deposited under accession number KCTC 14107BP.

In an embodiment, the first active material may additionally include a *Bifidobacterium animalis* subsp. *lactis* strain.

In an embodiment, the first active material may additionally include a *Bifidobacterium animalis* subsp. *lactis* strain and a *Lactobacillus acidophilus* strain.

In an embodiment, the strain may be lyophilized and included in an amount of 10³ to 10¹⁶ CFU/g. Specifically, for example, a single strain, a mixture of two strains, or a mixture of three strains may be included in an amount of 10³ to 10¹⁶ CFU/g, 10³ to 10¹⁵ CFU/g, 10³ to 10¹⁴ CFU/g, 10³ to 10¹³ CFU/g, 10³ to 10¹² CFU/g, 10⁴ to 10¹⁶ CFU/g, 10⁴ to 10¹⁵ CFU/g, 10⁴ to 10¹⁴ CFU/g, 10⁴ to 10¹³ CFU/g, 10⁴ to 10¹² CFU/g, 10⁵ to 10¹⁶ CFU/g, 10⁵ to 10¹⁵ CFU/g, 10⁵ to 10¹⁴ CFU/g, 10⁵ to 10¹³ CFU/g, 10⁵ to 10¹² CFU/g, 10⁶ to 10¹³ CFU/g, 10⁶ to 10¹² CFU/g, 10⁷ to 10¹³ CFU/g, 10⁷ to 10¹² CFU/g, 10⁸ to 10¹³ CFU/g, or 10⁸ to 10¹² CFU/g, but is not limited thereto.

For example, the composition according to an embodiment may contain 0.001 wt% to 80 wt% of the *Lactobacillus* sp. strain based on a total weight of the composition. In addition, an administration dose of the *Lactobacillus* sp. strain may be 0.01 mg to 10,000 mg, 0.1 mg to 1,000 mg, 1 mg to 100 mg, 0.01 mg to 1,000 mg, 0.01 mg to 100 mg, 0.01 mg to 10 mg, or 0.01 mg to 1 mg. The strain is included in the composition at a therapeutically effective amount or a nutritionally effective concentration, and for example, a single strain, a mixture of two strains, or a mixture of three strains may be included in a content of 10³ to 10¹⁶ CFU/g, 10³ to 10¹⁵ CFU/g, 10³ to 10¹⁴ CFU/g, 10³ to 10¹³ CFU/g, 10³ to 10¹² CFU/g, 10⁴ to 10¹⁶ CFU/g, 10⁴ to 10¹⁵ CFU/g, 10⁴ to 10¹⁴ CFU/g, 10⁴ to 10¹³ CFU/g, 10⁴ to 10¹² CFU/g, 10⁵ to 10¹⁶ CFU/g, 10⁵ to 10¹⁵ CFU/g, 10⁵ to 10¹⁴ CFU/g, 10⁵ to 10¹³ CFU/g, 10⁵ to 10¹² CFU/g, 10⁶ to 10¹³ CFU/g, 10⁶ to 10¹² CFU/g, 10⁷ to 10¹³ CFU/g, 10⁷ to 10¹² CFU/g, 10⁸ to 10¹³ CFU/g, or 10⁸ to 10¹² CFU/g, or may be included in the composition as a culture of an equivalent number of live cells or dead cells.

The therapeutically effective amount refers to an amount of a pharmaceutical composition containing the mixed strain and the herbal medicinal material; or the mixed strain and the herbal medicinal material; for the method and use of the present disclosure, the amount eliciting a biological or medical response or a desired therapeutic effect that a researcher, physician, or other clinician seeks to obtain in a patient. The therapeutically effective amount of the mixed strain and the herbal medicinal material may vary depending on factors such as a disease state, age, sex, and weight of an individual, and an ability of the herbal medicinal material to elicit a desired response in the individual. The therapeutically effective amount is also an amount in which therapeutically beneficial effects outweigh any toxic or detrimental effects.

In an embodiment, the mixed *Lactobacillus* sp. strain may have a sleep-inducing activity.

As used herein, the term "sleep disorder" refers to a disrupted sleep pattern caused by a plurality of causes including a dysfunctional sleep mechanism, an abnormality in physiological functions during sleep, an abnormality in a biological clock, and a sleep disorder induced by factors independent of a sleep process, and may be, for example, insomnia. The insomnia may include middle-of-the-night insomnia, late night insomnia, insomnia with prolonged wakefulness after sleep onset, sleep maintenance insomnia, and insomnia following middle-of-the-night awakenings.

As used herein, the term "sleep disorder-related disease" refers to a disease such as a defecation disorder, constipation, irritable bowel syndrome, depression, restless legs syndrome, myocardial infarction, or stroke, which is caused by physiological changes resulting from the sleep disorder.

In an embodiment, the second active material may include an herbal medicinal material. For example, the second active material may be at least one selected from the group consisting of Chineseyam, adlay, red ginseng, Reishi mushroom, and a combination thereof, but is not limited thereto.

As used herein, the term "herbal medicinal material" is defined in the Pharmaceutical Affairs Act as a raw medicinal material used to manufacture an herbal medicine or an herbal medicinal preparation. Since medicinal properties of the herbal medicinal material may vary depending on a traditional processing method, a processing method, a manufacturing method, and the like are important. In the present disclosure, the herbal medicinal material may be Chinese yam, adlay, red ginseng, or Reishi mushroom, but is not limited thereto. Here, the herbal medicinal material may be used in a form of a powder or an extract.

As used herein, the term "Chinese yam (*Dioscorea japonica*)" refers to a perennial vine plant of the order *Dioscoreales* and the family *Dioscoreaceae*, of which about 650 species in 10 genera are currently known, but about 10 species thereof are utilized as food resources. The Chinese yam mainly grows in mountainous areas, and is known to be distributed in regions such as Korea and Japan.

As used herein, the term "adlay (*Coix lachryma-jobi* var. *mayuen*)" refers to a plant belonging to the grass family, and is mainly cultivated in Southeast Asia. When used as an herbal medicinal material, the adlay is called Coix seed, and due to having diuretic, analgesic, and tonic actions, the Coix seed is used for edema, bladder stones, rheumatism, and the like. In addition, fresh leaves of the adlay are used as a tea substitute, and roots of the adlay are used to treat jaundice and neuralgia.

As used herein, the term "red ginseng" is a steamed root of ginseng (*Panax ginseng* C. A. Meyer). The red ginseng is known to have effects such as anti-aging, anti-fatigue and anti-stress effects, blood circulation improvement, an immune function, osteoporosis prevention, anemia treatment, a male infertility treatment effect, hypertension and diabetes alleviation, and cancer prevention. The red ginseng may be in a liquid or powder form.

As used herein, the term "Reishi mushroom" refers to *Ganoderma lucidum* (Leyss. ex. Fr) Karst, and is also called Seoncho, Gilsang mushroom, Jeokji, and the like. Because the Reishi mushroom is rich in minerals such as calcium and potassium and contains a large amount of unsaturated fatty acids, the Reishi mushroom is known to be effective against respiratory system diseases including phlegm removal and asthma relief, as well as adult disease prevention. In addition, efficacies of the Reishi mushroom in inhibiting cancer growth, lowering a cholesterol content in blood vessels, and lowering blood pressure are also known.

The herbal medicinal material of the present disclosure may be a mixture of one or more kinds.

Specifically, the herbal medicinal material may be a mixture of two kinds of herbal medicinal materials. In an embodiment, the herbal medicinal material may be a mixture of Chinese yam and adlay. In an embodiment, the herbal medicinal material may be a mixture of Chinese yam and red ginseng. In an embodiment, the herbal medicinal material may be a mixture of Chinese yam and Reishi mushroom. In an embodiment, the herbal medicinal material may be a mixture of adlay and red ginseng. In an embodiment, the herbal medicinal material may be a mixture of adlay and Reishi mushroom. In an embodiment, the herbal medicinal material may be a mixture of red ginseng and Reishi mushroom.

The herbal medicinal material may be a mixture of three kinds of herbal medicinal materials. In an embodiment, the herbal medicinal material may be a mixture of Chinese yam, adlay, and red ginseng. In an embodiment, the herbal medicinal material may be a mixture of Chinese yam, adlay, and Reishi mushroom. In an embodiment, the herbal medicinal material may be a mixture of Chinese yam, red ginseng, and Reishi mushroom. In an embodiment, the herbal medicinal material may be a mixture of adlay, red ginseng, and Reishi mushroom.

The herbal medicinal material may be a mixture of four kinds of herbal medicinal materials. Preferably, the four kinds of herbal medicinal materials of the Chinese yam, the adlay, the red ginseng, and the Reishi mushroom may be mixed at a predetermined ratio.

The herbal medicinal material may further include a substance other than the Chinese yam, the adlay, the red ginseng, and the Reishi mushroom.

As used herein, the term "culture" may be used interchangeably with a "culture supernatant", a "conditioned culture broth", or a "conditioned medium", and may refer to a whole medium including the *Lactobacillus* sp. strain, metabolites thereof, extra nutrients, and the like, which is obtained by culturing the strain for a certain period of time in a medium capable of supplying nutrients so that the strain can grow and survive *in vitro*. In addition, the culture broth may refer to a culture broth obtained by removing cells from a cell culture broth obtained by culturing the strain. The medium may be selected from known liquid media or solid media, and may be, for example, an MRS liquid medium, a GAM liquid medium, an MRS agar medium, a GAM agar medium, or a BL agar medium, but is not limited thereto.

In an embodiment, the composition for preventing or alleviating a sleep disorder may further contain a third active material including inulin or a vitamin; or a third active material may be further administered in combination.

In an embodiment, the third active material may be at least one selected from the group consisting of inulin, a vitamin, and a combination thereof, but is not limited thereto.

As used herein, the term "inulin" is a polysaccharide contained in large amounts in root vegetables, and is a kind of dietary fiber known as a fructan. The inulin is used as a means for storing energy in some plants. After ingestion, the inulin is not degraded by digestive enzymes in a body, but is fermented by intestinal microorganisms to promote a defecation function and is then excreted from the body. The inulin is known to have efficacies such as suppressing a postprandial blood sugar rise, improving cholesterol, and alleviating constipation.

As used herein, the term "vitamin" may be vitamin A, vitamin B (e.g., B1 or B2), vitamin C, vitamin E, vitamin K, or a combination thereof, but is not limited thereto.

The vitamin B2 is called riboflavin or lactoflavin, and is a kind of water-soluble vitamin. The vitamin B2 is contained in large amounts in liver, egg yolks, spinach, milk, and the like, and a deficiency thereof may cause symptoms such as growth retardation, premature aging, keratitis, dermatitis, alopecia, pharyngitis, and a gastrointestinal disorder.

In an embodiment, the composition may be one in which the first active material is *Lactobacillus fermentum* and *Lactobacillus plantarum*; the second active material is Chinese yam, adlay, red ginseng, and Reishi mushroom; the first active material additionally includes a *Bifidobacterium animalis* subsp. *lactis* strain and a *Lactobacillus acidophilus* strain; and the composition additionally contains inulin and a vitamin as the third active material.

As used herein, the term "contained as an active ingredient" means that the *Lactobacillus* sp. strain, a vesicle derived from the strain, a cell lysate of the strain, a culture broth, or an extract of the culture broth thereof is added, and encompasses being formulated into various forms by adding various components as secondary ingredients for drug delivery, stabilization, and the like.

In an embodiment, the composition may be one in which the first active material, the second active material, and the third active material are administered in combination simultaneously, sequentially, or in a reverse order.

As used herein, the terms "co-administration", "combination therapy", "combined treatment", or "in combination" may be used interchangeably, and refer to concurrent or parallel treatment of any form using at least two distinct materials useful for treating a disease. Components of the co-administration may be administered simultaneously, sequentially, in a reverse order, or in any order. The components may be administered in an appropriate manner at different doses, at different administration frequencies, or via different routes.

As used herein, the term "administration" refers to introducing a predetermined material into an individual by an appropriate method.

As used herein, the term "administered simultaneously" is not particularly limited, and means that the components of the co-administration are administered substantially simultaneously, for example, as a mixture or in an immediately consecutive sequence.

As used herein, the term "administered sequentially" is not particularly limited, and means that the components of the co-administration are not administered simultaneously, but are administered one by one in sequence or in groups with a specific time interval between administrations. The time interval may be the same or different between respective administrations of the components of the co-administration, and may be selected, for example, from a range of 2 minutes to 96 hours, 1 day to 7 days, or 1 week, 2 weeks, or 3 weeks. Generally, the time interval between administrations may range from several minutes to several hours, for example, 2 minutes to 72 hours, 30 minutes to 24 hours, or 1 to 12 hours. Additional examples include time intervals ranging from 24 to 96 hours, 12 to 36 hours, 8 to 24 hours, and 6 to 12 hours.

In an embodiment, the composition includes a first oral preparation containing the first active material, a second oral preparation containing the second active material, and a third oral preparation containing the third active material, and the first, second, and third oral preparations may be orally administered.

In an embodiment, the oral preparation may be in a form of tablets, pills, capsules, lozenges, granules, powders, suspensions, sachets, or syrups.

As used herein, the term "prevention" may refer to any act that suppresses a disease state of an individual or delays an onset thereof by administration of the pharmaceutical composition according to one aspect.

As used herein, the term "alleviation" may refer to any act that at least reduces a parameter, for example, a degree of a symptom, associated with a condition being treated.

As used herein, the term "treatment" may refer to any act in which a symptom regarding a disease state of an individual is ameliorated or beneficially altered by administration of the pharmaceutical composition according to one aspect.

Another aspect provides a health functional food for preventing or alleviating a sleep disorder, containing, as active ingredients, a first active material including a *Lactobacillus* sp. strain or a culture thereof and a second active material including an herbal medicinal material; or containing the first active material as an active ingredient, wherein the second active material is administered in combination.

Another aspect provides a health functional food for preventing or alleviating a defecation disorder or depression, containing, as active ingredients, a first active material including a *Lactobacillus* sp. strain or a culture thereof and a second active material including an herbal medicinal material; or containing the first active material as an active ingredient, wherein the second active material is administered in combination.

In an embodiment, co-administration of the mixed *Lactobacillus* sp. strain and/or the herbal medicinal material may alleviate a defecation disorder.

In an embodiment, co-administration of the mixed *Lactobacillus* sp. strain and/or the herbal medicinal material may alleviate depression.

As used herein, the term "defecation disorder" refers to a disease occurring in a lower rectum, an anus, and perianal tissues, and may refer to hemorrhoids, an anal fistula, an anal fissure, proctitis, pruritus ani, a rectal prolapse, or constipation, and the "constipation" may refer to a state in which two or more of the following symptoms persist for 3 months or longer: a case where a defecation frequency is twice or less per week, a case where a fecal weight is 35 g or less per day, or a case where straining during defecation, lumpy or hard stools, or a feeling of incomplete evacuation is present in 25 % or more of defecations.

As used herein, the term "depression" refers to a psychiatric disease that brings about a decline in daily functions by inducing various cognitive, mental, or physical symptoms with decreased motivation and a depressed mood as main symptoms, makes daily life difficult due to an overall functional decline in thought processes, motivation, volition, behavior, sleep, and the like, and has a high correlation with a prevalence rate and a suicide rate.

In an embodiment, the defecation disorder or the depression may be caused by a sleep disorder, but is not limited thereto.

In an embodiment, the health functional food may further contain a third active material including inulin or a vitamin; or a third active material may be further administered in combination.

In an embodiment, the second active material may be at least one selected from the group consisting of Chinese yam, adlay, red ginseng, Reishi mushroom, and a combination thereof, but is not limited thereto.

In an embodiment, the first active material, the second active material, and the third active material may be administered in combination simultaneously, sequentially, or in a reverse order.

In an embodiment, the health functional food includes a first oral preparation containing the first active material, a second oral preparation containing the second active material, and a third oral preparation containing the third active material, and the first, second, and third oral preparations may be orally administered.

As used herein, the term "health functional food" refers to a food manufactured and processed by using a specific component as a raw material or by a method such as extracting, concentrating, purifying, or mixing a specific component contained in a food raw material for a purpose of health supplementation, and refers to a food designed and processed so that a bioregulatory function, such as biodefense, biological rhythm regulation, and disease prevention and recovery, by the component can be sufficiently exerted on a living body. The health functional food may perform a function related to prevention and alleviation of a sleep disorder and the like.

A kind of the food is not particularly limited. Examples of a food to which the composition may be added include a formulation selected from the group consisting of powders, granules, tablets, capsules, pills, gels, jellies, suspensions, emulsions, syrups, teabags, leached teas, chewing gums, candies, and health drinks, and include all health foods in a conventional sense.

The health functional food may contain a food-acceptable food supplementary additive, and may contain an appropriate carrier commonly used in manufacturing a health functional food.

As used herein, the term "food-acceptable" means exhibiting a property of having no toxicity to a cell or a human exposed to the composition .

The health functional food may further include at least one of a carrier, a diluent, an excipient, and an additive, and may be formulated into one selected from the group consisting of a tablet, a pill, a powder, a granule, a fine powder, a capsule, and a liquid formulation. Foods to which the composition according to one aspect may be added include various foods, powders, granules, tablets, capsules, syrups, beverages, chewing gums, teas, vitamin complexes, health functional foods, and the like.

The health functional food may contain other components as an essential component without particular limitation, in addition to containing the active ingredient. For example, like a conventional beverage, the health functional food may contain various flavoring agents, natural carbohydrates, or the like as an additional component. Examples of the above-described natural carbohydrate may be conventional sugars such as a monosaccharide (e.g., glucose, fructose, etc.); a disaccharide (e.g., maltose, sucrose, etc.); and a polysaccharide (e.g., dextrin, cyclodextrin, etc.), and a sugar alcohol such as xylitol, sorbitol, and erythritol. As a flavoring agent other than those described above, a natural flavoring agent (thaumatin, a stevia extract (e.g., rebaudioside A, glycyrrhizin, etc.)) and a synthetic flavoring agent (saccharin, aspartame, etc.) may be advantageously used. A ratio of the natural carbohydrate may be appropriately determined by selection of those skilled in the art.

In addition to the above, the health functional food according to one aspect may contain various nutrients, vitamins, minerals (electrolytes), flavoring agents such as a synthetic flavoring agent and a natural flavoring agent, colorants and enhancers (cheese, chocolate, etc.), pectic acid and a salt thereof, alginic acid and a salt thereof, an organic acid, a protective colloidal thickener, a pH adjuster, a stabilizer, a preservative, glycerin, an alcohol, a carbonating agent used in carbonated beverages, and the like. These components may be used independently or in combination, and a ratio of these additives may also be appropriately selected by those skilled in the art.

In the health functional food, the active ingredient may be added to a food as it is, or may be used together with another food or a food component, and may be appropriately used according to a conventional method. A mixing amount of the active ingredient may be appropriately determined according to an intended use thereof (for prevention or alleviation). Generally, in manufacturing a food or a beverage, the health functional food may be added in an amount of specifically about 15 wt% or less, and more specifically about 10 wt% or less, relative to a raw material. However, in a case of long-term ingestion for a purpose of health and hygiene or for a purpose of health control, the amount may be below the above range.

Another aspect provides a food composition for preventing or alleviating a sleep disorder, a defecation disorder, or depression, containing, as active ingredients, a first active material including a *Lactobacillus* sp. strain or a culture thereof and a second active material including an herbal medicinal material; or containing the first active material as an active ingredient, wherein the second active material is administered in combination.

The food composition for preventing or alleviating a sleep disorder, a defecation disorder, and depression includes all forms such as a functional food, a nutritional supplement, a health food, and a food additive, and the food composition of this type may be manufactured into various forms according to a conventional method known in the art. For example, the composition of the present disclosure may be considered a food supplement. The food supplement, also known as a dietary supplement or a nutritional supplement, may be considered another specific pharmaceutical product. The food supplement is prepared for a purpose of supplementing a diet, and is intended to provide nutrients or beneficial ingredients that may not be ingested or may not be ingested in a sufficient amount from a normal diet. Most food supplements are considered foods, but are sometimes considered drugs, natural health products, or nutraceutical products. In the meaning of the present disclosure, the food supplement encompasses a health functional food. The food supplement is generally sold over the counter without a prescription. When the food supplement takes a form of a pill or a capsule, the food supplement includes the same excipients used in a pharmaceutical product. However, the food supplement may also take a form of a food fortified with some nutrients (e.g., infant formulas). Therefore, in a specific embodiment, the composition of the present disclosure is a food supplement.

In addition, the composition of the present disclosure may be included in various edible products and food products, such as milk products for infants. As used herein, the term "edible product" includes, in a broad sense, a product of any form that can be ingested by an animal in any manner (e.g., a product acceptable to sensory organs). The term "food product" is understood as an edible product that supplies nutritional support to a body. Foods of particular interest are food supplements and infant formulas. The food preferably includes a carrier material such as oatmeal gruel, lactic acid fermented foods, resistant starch, dietary fibers, carbohydrates, proteins, and glycosylated proteins. In a specific embodiment, the bacterial cell of the present disclosure is homogenized with another component, such as cereals or powdered milk, to constitute an infant formula.

Another aspect provides a feed composition for preventing or alleviating a sleep disorder, a defecation disorder, or depression, containing, as active ingredients, a first active material including a *Lactobacillus* sp. strain or a culture thereof and a second active material including an herbal medicinal material; or containing the first active material as an active ingredient, wherein the second active material is administered in combination.

Here, the feed composition may further contain at least one strain selected from the group consisting of a *Bifidobacterium animalis* subsp. *lactis* strain and a *Lactobacillus acidophilus* strain, or a culture thereof. In addition, the composition may further contain inulin and vitamin B2.

The feed composition for preventing or alleviating a sleep disorder, a defecation disorder, or depression may be manufactured by adding the strain or the culture thereof and the herbal medicinal material in an appropriate effective concentration range according to various feed manufacturing methods known in the art, and the feed composition may be used as a feed additive composition for a purpose of preventing or alleviating a sleep disorder-related disease.

As used herein, the "feed" may refer to any natural or artificial diet, a meal, and the like, or a component of the meal, which is intended for or suitable for an animal to eat, ingest, and digest. A kind of the feed is not particularly limited, and a feed commonly used in the art may be used. Non-limiting examples of the feed include a plant-based feed such as grains, root and tuber crops, food processing by-products, algae, fibers, pharmaceutical by-products, fats and oils, starches, meals, or grain by-products; and an animal-based feed such as proteins, inorganic matters, fats and oils, minerals, single-cell proteins, zooplankton, or foods.

Another aspect provides a pharmaceutical composition for preventing or treating a sleep disorder, containing, as active ingredients, a first active material including a *Lactobacillus* sp. strain or a culture thereof and a second active material including an herbal medicinal material; or containing the first active material as an active ingredient, wherein the second active material is administered in combination.

Another aspect provides a pharmaceutical composition for preventing or treating a defecation disorder or depression, containing, as active ingredients, a first active material including a *Lactobacillus* sp. strain or a culture thereof and a second active material including an herbal medicinal material; or containing the first active material as an active ingredient, wherein the second active material is administered in combination.

As used herein, the term "pharmaceutical composition" refers to a mixture containing one or more active ingredients and various components that deliver or assist the same for a purpose of a specific treatment, prevention, or diagnosis.

The pharmaceutical composition may additionally contain a pharmaceutically acceptable diluent or carrier. The diluent may be lactose, corn starch, soybean oil, microcrystalline cellulose, mannitol, or a combination thereof. The carrier may be an excipient, a disintegrant, a binder, a lubricant, or a combination thereof. The excipient may be microcrystalline cellulose, lactose, low-substituted hydroxycellulose, or a combination thereof. The disintegrant may be carboxymethyl cellulose calcium, sodium starch glycolate, anhydrous calcium hydrogen phosphate, or a combination thereof. The binder may be polyvinylpyrrolidone, low-substituted hydroxypropyl cellulose, hydroxypropyl cellulose, or a combination thereof. The lubricant may be magnesium stearate, silicon dioxide, talc, or a combination thereof.

When the pharmaceutical composition is formulated, the pharmaceutical composition may be prepared by using a diluent or an excipient such as a commonly used lubricant, sweetener, flavoring agent, emulsifier, suspending agent, preservative, filler, extender, binder, wetting agent, disintegrant, and surfactant. A solid preparation for oral administration may include a tablet, a pill, a powder, a granule, a capsule, and the like, and the solid preparation may be prepared by mixing the composition with at least one excipient, for example, starch, calcium carbonate, sucrose, lactose, gelatin, or the like. In addition to a simple excipient, a lubricant such as magnesium stearate and talc may also be used. A liquid preparation for oral administration includes a suspension, a liquid for internal use, an emulsion, a syrup, and the like, and may contain various excipients, for example, a wetting agent, a sweetener, a flavoring agent, a preservative, and the like, in addition to water and liquid paraffin, which are commonly used simple diluents. A preparation for parenteral administration may include a sterilized aqueous solution, a non-aqueous solvent, a suspension, an emulsion, a freeze-dried preparation, and a suppository. As the non-aqueous solvent and the suspension, propylene glycol, polyethylene glycol, a vegetable oil such as olive oil, an injectable ester such as ethyl oleate, and the like may be used. As a base of the suppository, Witepsol, macrogol, Tween 61, cacao butter, laurin butter, glycerogelatin, and the like may be used, and when the pharmaceutical composition is manufactured in a form of an eye drop, a known diluent or excipient may be used.

In the pharmaceutical composition, a carbohydrate such as glucose, sucrose, or dextran, an antioxidant such as ascorbic acid or glutathione, a chelating agent, a low-molecular-weight protein, or another stabilizer may be used as a pharmaceutical agent to increase stability or absorbability.

In an embodiment, the pharmaceutical composition may be formulated into a dosage form for oral or parenteral administration. The dosage form for oral administration may be a granule, a powder, a liquid, a tablet, a capsule, a dry syrup, or a combination thereof. For parenteral administration, external application to skin or an injection method such as an intraperitoneal injection, an intrarectal injection, a subcutaneous injection, an intravenous injection, an intramuscular injection, or an intrathoracic injection may be selected.

In an embodiment, administration routes of the pharmaceutical composition include, but are not limited to, oral, intravenous, intramuscular, intraarterial, intramedullary, intrathecal, intracardiac, transdermal, subcutaneous, intraperitoneal, intranasal, enteral, topical, sublingual, or rectal routes.

The pharmaceutical composition is administered in a pharmaceutically effective amount. The term "pharmaceutically effective amount" refers to an amount sufficient to treat a disease at a reasonable benefit/risk ratio applicable to a medical treatment, and an effective dose level may be determined according to factors including a kind of a disease of a patient, severity, an activity of a drug, sensitivity to the drug, an administration time, an administration route and an excretion rate, a treatment period, a concurrently used drug, and other factors well known in the medical field.

In one aspect, the pharmaceutical composition may be administered once a day, or may be administered in multiple divided doses. Specifically, the pharmaceutical composition may be administered in a cycle of 6 days of administration followed by 1 day of rest, 5 days of administration followed by 2 days of rest, or 4 days of administration followed by 3 days of rest per 7 days, and more specifically, may be administered in a cycle of 5 days of administration followed by 2 days of rest.

In addition, the pharmaceutically effective amount and an effective administration dose of the pharmaceutical composition may vary depending on a formulation method, an administration method, an administration time, and/or an administration route of the pharmaceutical composition. In addition, the pharmaceutically effective amount and the effective administration dose may vary depending on various factors including a kind and degree of a response to be achieved by administration of the pharmaceutical composition; a kind, age, weight, and general health state of an individual to be administered; a symptom or severity of a disease; sex, diet, and excretion; a component of a drug or another composition used concurrently or at different times in the individual; and similar factors well known in the medical field. A dose effective for a desired treatment can be easily determined and prescribed by a person having ordinary skill in the art. The pharmaceutical composition according to the present disclosure may be administered once a day, or may be administered in multiple divided doses. Therefore, the dose does not limit the scope of the present disclosure in any aspect. An administration dose of the pharmaceutical composition may be 1 µg/kg/day to 1,000 mg/kg/day.

Another aspect provides a method of preventing or alleviating a sleep disorder, including administering, to a subject in need thereof, a composition containing, as active ingredients, a first active material including a *Lactobacillus* sp. strain or a culture thereof and a second active material including an herbal medicinal material.

Another aspect provides a use of a composition for preventing or alleviating a sleep disorder, the composition containing, as active ingredients, a first active material including a *Lactobacillus* sp. strain or a culture thereof and a second active material including an herbal medicinal material.

Another aspect provides a use of a composition for the manufacture of a medicament for preventing or alleviating a sleep disorder, the composition containing, as active ingredients, a first active material including a *Lactobacillus* sp. strain or a culture thereof and a second active material including an herbal medicinal material.

Another aspect provides a method of preventing or alleviating a sleep disorder, including: administering a first active material including a *Lactobacillus* sp. strain or a culture thereof to a subject in need thereof; and administering a second active material including an herbal medicinal material to the subject in need thereof.

Another aspect provides a use of a composition for preventing or alleviating a sleep disorder, the composition containing a first active material including a *Lactobacillus* sp. strain or a culture thereof as an active ingredient, wherein a second active material including an herbal medicinal material is administered in combination.

Another aspect provides a use of a composition for the manufacture of a medicament for preventing or alleviating a sleep disorder, the composition containing a first active material including a *Lactobacillus* sp. strain or a culture thereof as an active ingredient, wherein a second active material including an herbal medicinal material is administered in combination.

Another aspect provides a use of a composition for preventing or alleviating a sleep disorder, the composition containing, as active ingredients, a first active material including a *Lactobacillus* sp. strain or a culture thereof and a second active material including an herbal medicinal material; or containing the first active material as an active ingredient, wherein the second active material is administered in combination.

Another aspect provides a use of a composition for the manufacture of a medicament for preventing or alleviating a sleep disorder, the composition containing, as active ingredients, a first active material including a *Lactobacillus* sp. strain or a culture thereof and a second active material including an herbal medicinal material; or containing the first active material as an active ingredient, wherein the second active material is administered in combination.

Another aspect provides a method of preventing, alleviating, or treating a sleep disorder-related disease, including administering, to a subject in need thereof, a composition containing, as active ingredients, a first active material including a *Lactobacillus* sp. strain or a culture thereof and a second active material including an herbal medicinal material.

Another aspect provides a use of a composition for preventing, alleviating, or treating a sleep disorder-related disease, the composition containing, as active ingredients, a first active material including a *Lactobacillus* sp. strain or a culture thereof and a second active material including an herbal medicinal material.

Another aspect provides a method of preventing, alleviating, or treating a sleep disorder-related disease, including: administering a first active material including a *Lactobacillus* sp. strain or a culture thereof to a subject in need thereof; and
administering a second active material including an herbal medicinal material to the subject in need thereof.

Another aspect provides a use of a composition for preventing or alleviating a sleep disorder, the composition containing a first active material including a *Lactobacillus* sp. strain or a culture thereof as an active ingredient, wherein a second active material including an herbal medicinal material is administered in combination.

Another aspect provides a method of preventing or alleviating a defecation disorder or depression, including administering, to a subject in need thereof, a composition containing, as active ingredients, a first active material including a *Lactobacillus* sp. strain or a culture thereof and a second active material including an herbal medicinal material.

Another aspect provides a use of a composition for preventing or alleviating a defecation disorder or depression, the composition containing, as active ingredients, a first active material including a *Lactobacillus* sp. strain or a culture thereof and a second active material including an herbal medicinal material.

Another aspect provides a method of preventing or alleviating a defecation disorder or depression, including: administering a first active material including a *Lactobacillus* sp. strain or a culture thereof to a subject in need thereof; and administering a second active material including an herbal medicinal material to the subject in need thereof.

Another aspect provides a use of a composition for preventing or alleviating a defecation disorder or depression, the composition containing a first active material including a *Lactobacillus* sp. strain or a culture thereof as an active ingredient, wherein a second active material including an herbal medicinal material is administered in combination.

The terms, methods, and the like described with respect to the present disclosure apply equally among the respective aspects.

Overlapping contents are omitted in consideration of complexity of the present specification, and terms not otherwise defined herein have meanings commonly used in the art to which the present disclosure pertains.

### Advantageous Effects of Invention

According to the composition for preventing or alleviating a sleep disorder, in which a *Lactobacillus* sp. strain and an herbal medicinal material are administered in combination according to one aspect, the composition may be useful for preventing or treating a sleep disorder-related disease, such as alleviating a defecation disorder symptom induced by a sleep disorder or reducing depression induced by a sleep disorder.

### Brief Description of Drawings

FIG. 1 shows a change in circadian rhythm gene expression according to co-administration of a mixed strain and an herbal medicinal material according to an embodiment.
FIG. 2 is a diagram showing a process of recruiting research participants to participate in a human application test process of co-administration of a mixed strain and an herbal medicinal material according to an embodiment.

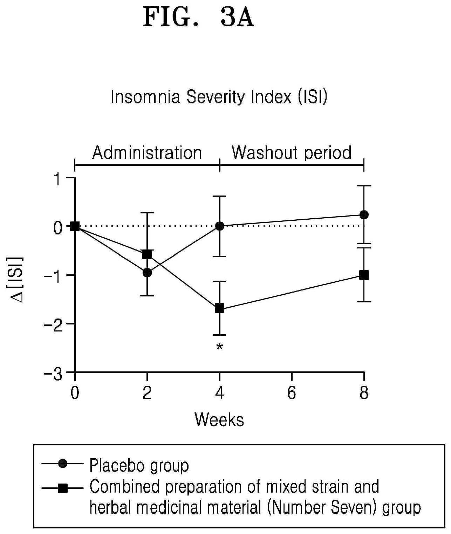

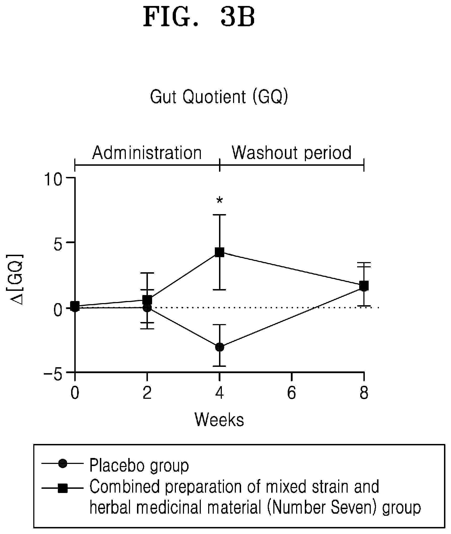

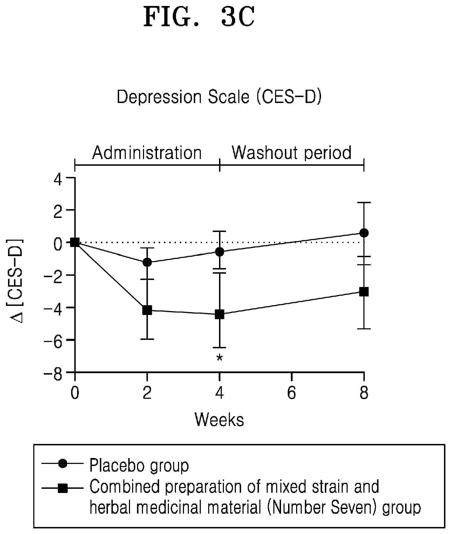

FIG. 3 is a graph showing results of a human application test of co-administration of a mixed strain and an herbal medicinal material according to an embodiment: FIG. 3A is a graph showing results of measuring a change in an Insomnia Severity Index (ISI) of a group taking a combined preparation of a mixed strain and an herbal medicinal material (Number Seven) and a group taking a placebo according to an embodiment; FIG. 3B is a graph showing results of evaluating a change in a Gut Quotient (GQ) of the group taking the combined preparation of the mixed strain and the herbal medicinal material (Number Seven) and the group taking the placebo according to an embodiment; and FIG. 3C is a graph showing results of evaluating a change in CES-D of the group taking the combined preparation of the mixed strain and the herbal medicinal material (Number Seven) and the group taking the placebo according to an embodiment.

### Mode for the Invention

Hereinafter, preferred examples are provided to aid in understanding the present disclosure. However, the following examples are provided only to more easily understand the present disclosure, and the contents of the present disclosure are not limited by the following examples. Since various modifications may be made to the examples, the examples are not limited to the examples disclosed below, but may be implemented in various forms.

### Example 1. Isolation and identification of Lactobacillus fermentum and Lactobacillus plantarum strains

### 1.1. Isolation of strains

The Lactobacillus *fermentum* strain and the *Lactobacillus plantarum* strain of the present disclosure were isolated from a vaginal sample of a healthy woman who visited a hospital for a health checkup. Specifically, an internal vaginal sample was collected with a cotton swab, streak-inoculated onto a Rogosa SL (MRS) agar plate, and cultured in an anaerobic chamber at 37 °C for 48 hours. When bacterial colonies grew, single colonies were subcultured onto a fresh MRS agar plate for pure isolation. After the pure isolation, the strains were cultured using an MRS medium.

### 1.2. Selection of strains having an anti-adipogenic activity

To select a strain having an anti-adipogenic activity, an inhibitory activity against a pancreatic lipase and an ability to inhibit differentiation of 3T3-L1 preadipocytes into adipocytes were confirmed.

Specifically, the inhibitory activity against the pancreatic lipase was confirmed by diluting the strain of 1.1 above to a concentration of 0.1 mg/mL, placing the diluted strain in a plate together with a 0.167 mM p-nitrophenyl palmitate (PNP; Sigma, USA) solution, a 0.061 M Tris-HCl buffer (pH 8.5), and a 0.3 mg/mL lipase solution, allowing the resulting mixture to react at 25 °C for 10 minutes, and then measuring an absorbance at 405 nm.

In addition, the ability to inhibit the differentiation of the 3T3-L1 preadipocytes into the adipocytes was measured using Oil Red O (Sigma, USA), which specifically reacts with lipid droplets generated in cells. After adipogenic differentiation was completed, a medium was removed, and then the cells were washed twice with PBS, fixed with 10 % formalin at 40 °C for 1 hour, washed twice with 60 % isopropanol, and stained with a 0.5 % Oil Red O solution at room temperature for 30 minutes. After the staining, a staining solution was removed, and the cells were washed twice using distilled water. Then, when the distilled water had completely dried, isopropyl alcohol was added, and then an absorbance was measured at 520 nm.

Finally, a 3T3-L1 cell viability for the strain obtained in 1.1 above was measured using an MTT (3-(4,5-dimethylthiazol-2-yl)-2,5-diphenyltetrazolium bromide) method. The 3T3-L1 cells were seeded in a 96-well plate at a concentration of 16 × 10⁴ cells/well, and after culturing for 24 hours, a medium was removed. Thereto, lactic acid bacteria samples diluted to concentrations of 100 and 1,000 µg/mL in 100 µL of a fresh DMEM medium were respectively added, and the cells were cultured for 24 hours. Thereafter, 20 µL of an MTT (Sigma, USA) solution prepared at 5 mg/mL was added, and the cells were cultured at 37 °C for 4 hours. After the culturing, a supernatant was removed, 200 µL of dimethyl sulfoxide (DMSO) was added, and then an absorbance was measured at 546 nm.

From the above results, an anti-adipogenic effect of each strain could be confirmed, and among the prepared strains, *Lactobacillus fermentum* GB102 (hereinafter referred to as 'GB102'), *Lactobacillus fermentum* GB103 (hereinafter referred to as 'GB103'), and *Lactobacillus plantarum* GB104 (hereinafter referred to as 'GB104') strains, which exhibited an adipocyte accumulation inhibitory effect and low cytotoxicity, were finally selected. In addition, the strain name of *Lactobacillus fermentum* has been changed to *Limosilactobacillus fermentum*, and the strain name of *Lactobacillus plantarum* has been changed to *Lactiplantibacillus plantarum*. In the following examples, the changed strain names of the existing strains are described interchangeably.

### 1.3. Molecular biological identification of selected strains

For identification of the finally selected *Lactobacillus fermentum* GB102 and GB103 strains and the *Lactobacillus plantarum* GB104 strain, the strains were analyzed using a 16S rRNA gene sequence. The 16S rRNA gene sequences obtained through PCR using 27F and 1492R primers targeting a bacterial 16S rRNA gene were analyzed by a Sanger sequencing method, and the 16S rRNA sequences of *Lactobacillus fermentum* GB102 and GB103 and *Lactobacillus plantarum* GB104 were represented as SEQ ID NOs: 1, 2, and 3, respectively.

In addition, a homology analysis thereof was performed, and as a result, as shown in Table 1 below, the GB102 and GB103 strains showed the highest sequence similarity to a type strain of *Limosilactobacillus fermentum*, and subsequently showed a high sequence similarity to *Limosilactobacillus gorillae*. GB104 showed a sequence similarity of 98 % or more with 18 species belonging to the genus *Lactiplantibacillus*, including a type strain of *Lactiplantibacillus plantarum*, based on the 16S rRNA sequence, and thus species could not be distinguished by the 16S rRNA gene sequence. Accordingly, it was confirmed through a comparison of Average Nucleotide Identity (ANI) values after whole-genome sequencing that the GB104 is a strain belonging to *Lactiplantibacillus plantarum*. In conclusion, it was confirmed that the two strains correspond to *Limosilactobacillus fermentum*, and it was confirmed that one strain corresponds to *Lactiplantibacillus plantarum*.

**[Table 1]**

| Strain | Species | Type strain name | Sequence similarity (%) |
|---|---|---|---|
| GB102 | *Limosilactobacillus fermentum* | CECT 562(T) | 99.79 |
| | *Limosilactobacillus gorillae* | KZ01(T) | 98.26 |
| GB103 | *Limosilactobacillus fermentum* | CECT 562(T) | 99.93 |
| | *Limosilactobacillus gorillae* | KZ01(T) | 98.13 |
| GB104 | *Lactiplantibacillus pentosus* | DSM 20314(T) | 99.93 |
| | *Lactiplantibacillus argentoratensis* | DSM 16365(T) | 99.87 |
| | *Lactiplantibacillus plantarum* | ATCC 14917(T) | 99.87 |
| | *Lactiplantibacillus paraplantarum* | DSM 10667(T) | 99.73 |
| | *Lactiplantibacillus paraplantarum* | DSM 10667(T) | 99.66 |
| | *Lactiplantibacillus herbarum* | TCF032-E4(T) | 98.92 |
| | *Lactiplantibacillus daoliensis* | 116-1A(T) | 98.82 |
| | *Lactiplantibacillus pingfangensis* | 382-1(T) | 98.81 |
| | *Lactiplantibacillus daowaiensis* | 203-3(T) | 98.75 |
| | *Lactiplantibacillus nangangensis* | 381-7(T) | 98.74 |
| | *Lactiplantibacillus plajomi* | NB53(T) | 98.72 |
| | *Lactiplantibacillus fabifermentans* | DSM 21115(T) | 98.72 |
| | *Lactiplantibacillus garii* | FI11369(T) | 98.61 |
| | *Lactiplantibacillus xiangfangensis* | LMG 26013(T) | 98.59 |
| | *Lactiplantibacillus modestisalitolerans* | NB466(T) | 98.52 |
| | *Lactiplantibacillus mudanjiangensis* | 11050(T) | 98.2 |
| | *Lactiplantibacillus songbeiensis* | 398-2(T) | 98.19 |
| | *Lactiplantibacillus dongliensis* | 218-3(T) | 98.12 |

### Example 2. Genomic and comparative genomic analysis of Lactobacillus fermentum strains

To investigate genome-based species identification and characteristics of the GB102 and GB103 strains, genome sequences of the strains were completely sequenced, and functions of genes included in the genomes were inferred by utilizing a next-generation sequencing (NGS) technology and a bioinformatics technology. In addition, the specificity of the strains was confirmed through a comparative analysis with completely sequenced genome sequences of the same species. The strains were cultured in an MRS liquid medium under an anaerobic condition at 37 °C for 4 hours, and genomic DNA was extracted from a culture using an MG Genomic DNA purification kit (MGMED, Inc., Korea). To obtain long read data having an average length of 10 kb or more, sequence analysis was performed using a PacBio RS II system, and to produce highly accurate short read data having a sequence length of less than 500 bp to complement the long read data having lower accuracy, sequence analysis was performed using a NovaSeq 6000 system. The long read data were assembled into highly complete GB102 and GB103 draft genomes utilizing an HGAP2 pipeline of an SMRT Analysis server. SNP and InDel errors that may be present in the assembled draft genome sequences were corrected through the long read data and the short read data. From the completed genome sequences, coding sequences (CDSs) were predicted using a Prodigal program, and rRNAs and tRNAs were predicted utilizing an Rfam tool. Functions of the predicted CDSs were predicted by performing a homology search (applying a BLAST algorithm) based on the publicly available UniProt, GenBank nr, Subsystem, Pfam and COG databases. An Average Nucleotide Identity (ANI) was calculated utilizing a JSpecies program from genome sequences of a type strain B1 28^{T} (= ATCC 14931^{T}) of a *Lactobacillus fermentum* species, a type strain KZ01^{T} of *Lactobacillus gorillae*, and a type strain DSM 16045^{T} of *Lactobacillus gastricus* published in GenBank, and the genome sequences of the GB102 and GB103 strains. As a result of the analysis, the two strains, GB102 and GB103, showed an ANI value of 95 % or more with the *Lactobacillus fermentum* type strain, proving that the two strains belong to the *Lactobacillus fermentum* species (Table 2). In addition, because the GB102 and GB103 strains do not 100 % match the type strain, it can be seen that the strains are novel strains that have not been previously isolated and reported. Furthermore, it was confirmed that the GB102 and GB103 strains are also different strains from each other because the genomic distance therebetween is large. The present inventors named the GB102 and GB103 strains "*Lactobacillus fermentum* GB102" (Accession No.: KCTC 14105BP) and "*Lactobacillus fermentum* GB103" (Accession No.: KCTC 14106BP), respectively, and deposited the strains at the Korean Collection for Type Cultures (KCTC) of the Korea Research Institute of Bioscience and Biotechnology on January 14, 2020.

**[Table 2]**

| Strain name | Genome size (bp) | GC ratio | ANI values | | | | |
|---|---|---|---|---|---|---|---|
| | | | GB102 | GB103 | B1 28 | KZ01 | DSM 16045 |
| GB102 | 2,039,432 | 51.88 | **-**-- | **98.43** | **99.14** | 79.31 | 68.77 |
| GB103 | 2,260,513 | 51.25 | **98.51** | **-**-- | **98.42** | 79.72 | 69.23 |
| B1 28 | 1,905,587 | 52.30 | **99.09** | **98.51** | **-**-- | 79.42 | 68.66 |
| KZ01 | 1,641,621 | 48.11 | 79.05 | 79.37 | 79.11 | --- | 68.07 |
| DSM 16045 | 1,848,461 | 41.64 | 68.22 | 68.38 | 68.02 | 68.18 | --- |

### Example 3. Genomic and comparative genomic analysis of Lactobacillus plantarum strain

To investigate genome-based species identification and characteristics of the GB104 strain, a genome sequence of the strain was completely sequenced, and functions of genes included in the genome were inferred by utilizing a next-generation sequencing (NGS) technology and a bioinformatics technology. In addition, the specificity of the strain was confirmed through a comparative analysis with completely sequenced genome sequences of the same species. The strain was cultured in an MRS liquid medium under an anaerobic condition at 37 °C for 4 hours, and genomic DNA was extracted from a culture using an MG Genomic DNA purification kit (MGMED, Inc., Korea). To obtain long read data having an average length of 10 kb or more, sequence analysis was performed using a PacBio RS II system, and to produce highly accurate short read data having a sequence length of less than 500 bp to complement the long read data having lower accuracy, sequence analysis was performed using a NovaSeq 6000 system. The long read data were assembled into a highly complete GB104 draft genome utilizing an HGAP2 pipeline of an SMRT Analysis server. SNP and InDel errors that may be present in the assembled draft genome sequence were corrected through the long read data and the short read data. From the completed genome sequence, coding sequences (CDSs) were predicted using a Prodigal program, and rRNAs and tRNAs were predicted utilizing an Rfam tool. Functions of the predicted CDSs were predicted by performing a homology search (applying a BLAST algorithm) based on the publicly available UniProt, GenBank nr, Subsystem, Pfam, and COG databases. The genome of GB104 is summarized and shown in Table 3.

**[Table 3]**

| Features | Values | % |
|---|---|---|
| Genome size (bp) | 3,247,930 | 100.00 |
| DNA coding (bp) | 2,742,575 | 84.44 |
| DNA G+C (bp) | 1,445,117 | 44.49 |
| Total genes | 3,087 | 100.00 |
| Protein coding genes | 2,990 | 96.86 |
| Genes with function prediction | 2,517 | 81.54 |
| RNA genes | 87 | 2.82 |
| rRNA genes | 16 | 0.52 |
| tRNA genes | 68 | 2.20 |
| Pseudo genes | 10 | 0.32 |

As a result of the genome analysis, the strain possessed a plantaricin gene cluster, which is a bacteriocin biosynthesis gene cluster related to an antibacterial activity found in *Lactobacillus plantarum*. However, because the strain does not 100 % match a type strain and a reference strain, it can be seen that the strain is a novel strain that has not been previously isolated and reported.

The present inventors named the GB104 strain "*Lactobacillus plantarum* GB104" (Accession No.: KCTC 14107BP) and deposited the strain at the Korean Collection for Type Cultures (KCTC) of the Korea Research Institute of Bioscience and Biotechnology on January 14, 2020.

### Example 4. Complex preparation of mixed strain and herbal medicinal material

The complex preparation of the mixed strain and the herbal medicinal material was prepared by additionally incorporating inulin and vitamin B2 into a mixture of the mixed strain consisting of the *Lactobacillus* sp. strains and the herbal medicinal material.

Specifically, the mixed strain consisting of the *Lactobacillus* sp. strains was prepared by mixing three kinds of lactic acid bacteria, GB102, GB103, and GB104, and Andong Chinese yam, adlay, red ginseng powder, and Reishi mushroom were used as the herbal medicinal material.

The mixed strain obtained by mixing the three kinds of lactic acid bacteria, GB102, GB103, and GB104, was named #7 lactic acid bacteria or No. 7 lactic acid bacteria, and the complex preparation of the mixed strain and the herbal medicinal material was named #7 or No. 7.

### Experimental Example 1. Confirmation of changes in circadian rhythm gene expression according to co-administration of mixed strain and herbal medicinal material

Aging causes a change in a circadian rhythm, which may affect a sleep disorder, a defecation disorder, and depression. Due to aging, expression of BMAL1 and CLOCK and a regulatory mechanism through REV-ERBα/β and RORα/γ proteins are weakened, and regulatory functions of PER and CRY proteins are decreased, which may result in a decrease in sleep quality and frequent awakenings. In addition, as regulation of expression of a gene binding to a D-Box and an E-Box changes, a defecation disorder such as constipation and irritable bowel syndrome (IBS) may be induced, and these changes may degrade a quality of life of an elderly person, thereby increasing risks of depression, cognitive decline, and frailty.

To confirm that a negative effect of the circadian rhythm change caused by aging on physical and mental health can be improved by administration of the complex preparation of the mixed strain and the herbal medicinal material (Number Seven) of Example 4, the complex preparation of the mixed strain and the herbal medicinal material (Number Seven) of Example 4 or PBS was orally administered to aged mice (16 months old) daily for 8 months, and corresponding tissue (intestinal and muscle tissue) samples were collected from the aged test subject mice, and a gene expression pattern was analyzed through RNA sequencing. Young mice aged 9 to 10 weeks were used as a control group, and a gene expression pattern was also analyzed for mice orally administered with only probiotics (*Limosilactobacillus fermentum*, *Lactiplantibacillus plantarum*, *Bifidobacterium animalis* subsp.*,* and *Lactobacillus acidophilus*: Number Seven mixed strain) or prebiotics (inulin, fructooligosaccharide, Chinese yam, dehulled adlay, red ginseng, and Reishi mushroom) included in the complex preparation of the mixed strain and the herbal medicinal material (Number Seven) during the same period.

A specific experimental process is as follows.

### 1.1. Performance of NGS

For RNA-Seq analysis of 5 kinds of tissues of 5 kinds of experimental group mice conducted in 5 replicates, corresponding tissue samples were collected from the test subject mice, and an RNA preservative (RNAlater, USA) was added thereto immediately, and the samples were stored at -80 °C. Thereafter, for RNA extraction, each sample was thawed, and then treated with DNase to degrade and remove DNA molecules, and a strand-specific RNA sequencing library was constructed after selectively isolating and purifying mRNA molecules from the obtained total RNA by applying a TruSeq Stranded mRNA Library Prep Kit. Construction of the sequencing library was performed by sequentially going through processes of random fragmentation of the extracted RNA, synthesis of cDNA fragments through reverse transcription, attachment of sequencing adapters to both ends of the cDNA fragments followed by PCR, and size selection for securing cDNA fragments having a length of 200 to 400 bp, and paired-end sequencing of the constructed final sequencing library was performed at a length of 100 bp × 2 using a NovaSeq 6000 sequencer of an Illumina sequencing platform.

### 1.2. Bioinformatics analysis

For raw RNA-Seq sequence sets of a total of 125 samples obtained through the performance of the NGS, sequence preprocessing, reference genome mapping, and gene expression quantification processes were sequentially performed to obtain a gene expression profile for each sample, and reproducibility of the gene expression profile in replicates and relative similarity between the experimental groups were evaluated through a hierarchical clustering analysis and a principal coordinate analysis (PCoA). Statistically significantly differentially expressed genes (DEGs) were selected by performing a statistical test between comparison conditions on gene expression values, and to more effectively understand functional roles of the selected differentially expressed gene sets, functional categories to which the differentially expressed genes statistically significantly belong were analyzed at a gene set level including Gene Ontology and metabolic pathways, and complementarily, the differentially expressed gene sets were analyzed and reviewed together through an expression comparison at a direct functional category level. In the sequence preprocessing, a sequencing adapter sequence portion and a low-quality nucleotide sequence portion were trimmed off by applying a Trimmomatic program (Bolger, et al., 2014), and the reference genome mapping was performed on a mouse reference genome sequence (Mus musculus reference genome, mm39) by applying a HISAT2 program (Kim, et al., 2019). In the gene expression quantification, gene expression levels were obtained in units of Fragments Per Kilobase Million (FPKM) and Transcripts Per Million (TPM) by applying a StringTie program (Pertea, et al., 2015), and the differentially expressed genes were selected by applying a Ballgown program (Pertea, et al., 2016). A functional category analysis of the differentially expressed genes was performed by applying a g:Profiler program (Kolberg, et al., 2023), and an analysis of the differentially expressed gene sets at the direct functional category level was performed by applying a Gene Set Enrichment Analysis (GSEA) program (Subramanian, et al., 2005).

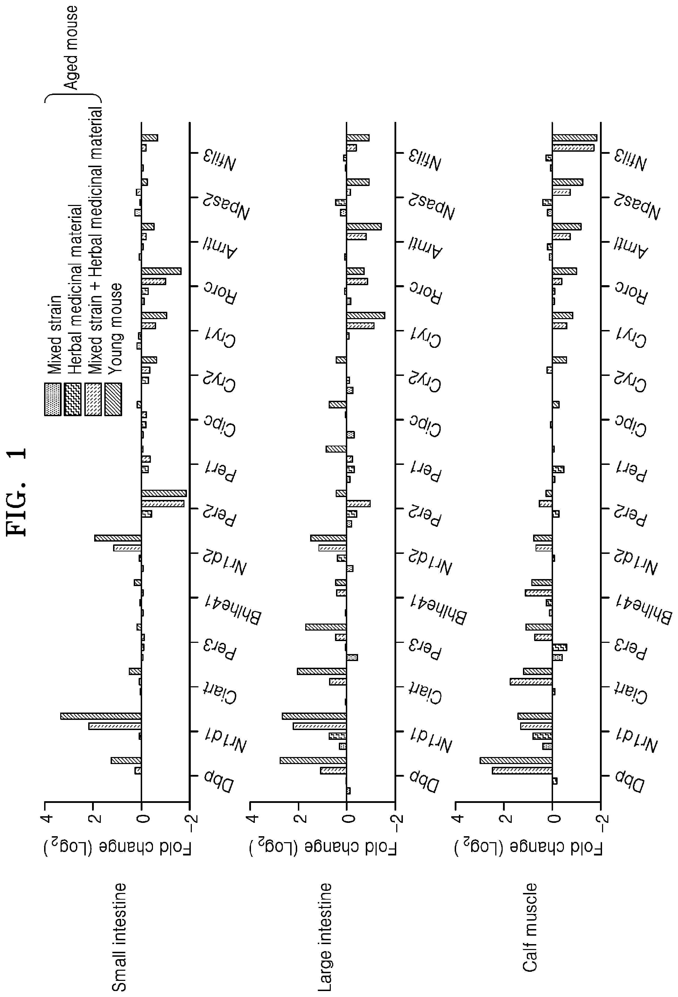

A result of confirming the gene expression pattern analyzed through the above process is shown in FIG. 1.

FIG. 1 shows a change in circadian rhythm gene expression according to co-administration of a mixed strain and an herbal medicinal material according to an embodiment.

As shown in FIG. 1, when the complex preparation of the mixed strain and the herbal medicinal material (Number Seven) of the present disclosure was administered, it was confirmed that an expression pattern of a gene related to a circadian rhythm change showed a pattern similar to that of the young mice.

These results mean that the complex preparation of the mixed strain and the herbal medicinal material (Number Seven) of the present disclosure participates in an expression pattern of a circadian-related gene, thereby suppressing a weakening of a circadian rhythm caused by aging.

### Experimental Example 2. Human application test of co-administration of mixed strain and herbal medicinal material

To confirm a sleep improvement effect, a defecation disorder improvement effect, and a depression improvement effect when taking the complex preparation of the mixed strain and the herbal medicinal material (Number Seven) of Example 4, a human application test was conducted.

### 2.1. Selection of test subjects and selection of final analysis subjects

A total of 65 adults aged 60 or older were inquired about the possibility of participation in the human application test, and 60 individuals were recruited, excluding those who did not meet the selection criteria (2 individuals with underlying diseases and 3 individuals who refused to participate). The individuals were randomly assigned into a group taking the complex preparation of the mixed strain and the herbal medicinal material (Number Seven) and a placebo taking group, 30 individuals per group, to start medication, and dropouts due to a simple change of mind and antibiotic use occurred in 3 individuals in the placebo taking group and 6 individuals in the group taking the complex preparation of the mixed strain and the herbal medicinal material (Number Seven). At a 3rd visit (4 weeks of medication), a questionnaire survey on medication was conducted, and medication compliance was evaluated by collecting a remaining preparation after medication from each subject. Subjects having a medication compliance of less than 80 % were additionally excluded, which occurred in 1 individual in the placebo taking group and 2 individuals in the group taking the complex preparation of the mixed strain and the herbal medicinal material (Number Seven), and thus an effect of taking the complex preparation of the mixed strain and the herbal medicinal material (Number Seven) was evaluated with results of the final 26 individuals in the placebo taking group and the final 22 individuals in the group taking the complex preparation of the mixed strain and the herbal medicinal material (Number Seven).

During the random assignment, the subjects were assigned such that a sex ratio was homogeneous between both groups, and the analysis was performed without including other correction variables on the premise that other factors would be homogeneous between both groups.

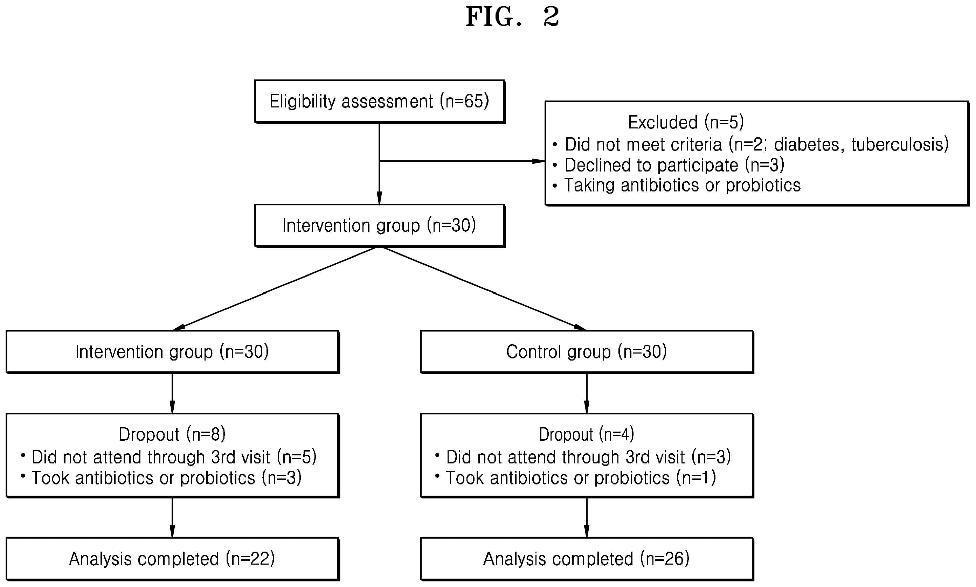

Specific details on the selection and the analysis of the test subjects are shown in Table 4 and FIG. 2 below. FIG. 2 shows a process of recruiting research participants to participate in a human application test process of co-administration of a mixed strain and an herbal medicinal material according to an embodiment.

**[Table 4]**

| **Characteristics** | **Total (n=48)** | | **Complex preparation of mixed strain and herbal medicinal material (Number Seven) (n=22)** | | **Placebo (n=26)** | | P^{*} |
|---|---|---|---|---|---|---|---|
| | **N** | **(%)** | **N** | **(%)** | **N** | **(%)** | |
| **Sex** | | | | | | | |
| Male | 14 | (29.2) | 5 | (22.7) | 9 | (34.6) | 0.171 |
| Female | 34 | (70.8) | 17 | (77.3) | 17 | (65.4) | |

| **Age (years)** | | | | | | | |
|---|---|---|---|---|---|---|---|
| 60-69 | 13 | (27.1) | 6 | (27.3) | 7 | (26.9) | 0.918 |
| 70-79 | 28 | (58.3) | 12 | (54.6) | 16 | (61.5) | |
| 80+ | 7 | (14.6) | 4 | (18.2) | 3 | (11.5) | |

| **Highest level of education** | | | | | | | |
|---|---|---|---|---|---|---|---|
| Elementary school | 18 | (37.5) | 6 | (27.3) | 12 | (46.2) | 0.367 |
| Middle or high school | 20 | (41.7) | 11 | (50.0) | 9 | (34.6) | |
| graduate | | | | | | | |
| College graduate or higher | 10 | (20.8) | 5 | (22.7) | 5 | (19.2) | |

| **Cohabitant** | | | | | | | |
|---|---|---|---|---|---|---|---|
| Yes | 32 | (66.7) | 13 | (59.1) | 19 | (73.1) | 0.306 |
| No | 16 | (33.3) | 9 | (40.9) | 7 | (26.9) | |

| **Monthly income (KRW)** | | | | | | | |
|---|---|---|---|---|---|---|---|
| < 500,000 | 14 | (29.2) | 6 | (27.3) | 8 | (30.8) | 0.374 |
| 500,000∼1,999,999 | 17 | (35.4) | 10 | (45.5) | 7 | (26.9) | |
| ≥2,000,000 | 17 | (35.4) | 6 | (27.3) | 11 | (42.3) | |

| **Smoking status** | | | | | | | |
|---|---|---|---|---|---|---|---|
| Non-smoker | 39 | (81.3) | 18 | (81.8) | 21 | (80.8) | 0.999 |
| Former smoker | 7 | (14.6) | 3 | (13.6) | 4 | (15.4) | |
| Current smoker | 2 | (4.2) | 1 | (4.6) | 1 | (3.9) | |

| **Drinking status** | | | | | | | |
|---|---|---|---|---|---|---|---|
| Yes | 27 | (56.3) | 10 | (45.5) | 17 | (65.4) | 0.166 |
| No | 21 | (43.8) | 12 | (54.6) | 9 | (34.6) | |

| **Physical activity** | | | | | | | |
|---|---|---|---|---|---|---|---|
| Yes | 19 | (39.6) | 8 | (36.4) | 11 | (42.3) | 0.675 |
| No | 29 | (60.4) | 14 | (63.6) | 15 | (57.7) | |

| **Chronic disease** | | | | | | | |
|---|---|---|---|---|---|---|---|
| No | 18 | (37.5) | 7 | (31.8) | 11 | (42.3) | 0.455 |
| Yes | 30 | (62.5) | 15 | (68.2) | 15 | (57.7) | |
| Hypertension | 24 | (50.0) | 14 | (63.6) | 10 | (38.4) | |
| Diabetes | 2 | (4.2) | 2 | (9.1) | 0 | (0) | |
| Hyperlipidemia | 17 | (35.4) | 10 | (45.5) | 7 | (26.9) | |

| | | | | | | | |
|---|---|---|---|---|---|---|---|
| * Chi-square test was performed. | | | | | | | |

### 2.2. Study design

This study was conducted as a double-blind, randomized, controlled trial (RCT) divided into a complex preparation of the mixed strain and the herbal medicinal material (Number Seven) group and a placebo group. The complex preparation of the mixed strain and the herbal medicinal material (Number Seven) was mainly composed of probiotics (*Limosilactobacillus fermentum, Lactiplantibacillus plantarum, Bifidobacterium animalis* subsp.*,* and *Lactobacillus acidophilus*) and prebiotics (inulin, fructooligosaccharide, Chinese yam, dehulled adlay, red ginseng, and Reishi mushroom), and was provided in three capsules. The complex preparation of the mixed strain and the herbal medicinal material (Number Seven) was prepared to include at least 5 × 10¹⁰ CFU (colony-forming units) of the mixed strain. In the group taking the complex preparation of the mixed strain and the herbal medicinal material (Number Seven), the three capsules were taken daily for 4 weeks, and in the placebo taking group, three capsules mainly containing maltodextrin were taken daily for 4 weeks. All the participants were also followed up 4 weeks after stopping the administration of the complex preparation of the mixed strain and the herbal medicinal material (Number Seven) or the placebo. At weeks 0, 2, 4, and 8, blood and fecal sample collection, physical ability measurement, and a questionnaire survey were performed, and all measurement procedures were conducted at Seoul National University College of Medicine.

### 2.3. Questionnaire survey

Demographic information such as age, sex, education level, marital status, income, and occupational status of the participants, and behavioral characteristics such as smoking and drinking were collected through a questionnaire. In addition, a diagnosis history and a treatment status of chronic diseases including hypertension, diabetes, and dyslipidemia were also collected.

### Experimental Example 3. Confirmation of effect of co-administration therapy of mixed strain and herbal medicinal material on sleep improvement

To confirm a sleep disorder improvement effect upon co-administration of the mixed strain consisting of the *Lactobacillus* sp. strains and the herbal medicinal material, an Insomnia Severity Index (ISI) evaluating a sleep quality of the participants recruited through the process of Experimental Example 2 was evaluated.

The Insomnia Severity Index (ISI) is a self-report questionnaire tool consisting of seven items regarding sleep onset, sleep maintenance, early morning awakening problems, sleep dissatisfaction, an impact of sleep problems on daytime functioning, noticeability of sleep problems by others, and distress caused by sleep problems, and a higher ISI score indicates lower sleep quality.

The Insomnia Severity Index (ISI) is a simple yet validated tool, and is useful to measure severity of insomnia and evaluate an effect of an insomnia treatment, and reliability and validity of a Korean version of the ISI have been demonstrated through a study verifying the same.

Results of measuring the Insomnia Severity Index of the group taking the complex preparation of the mixed strain and the herbal medicinal material (Number Seven) and the placebo taking group according to Experimental Example 2.2 are shown in Table 5 and FIG. 3A.

**[Table 5]**

| Measure | Placebo | | | Complex preparation of mixed strain and herbal medicinal material (Number Seven) | | |
|---|---|---|---|---|---|---|
| | Week 0 Mean ± standard deviation | Week 4 Mean ± standard deviation | *P*-value | Week 0 Mean ± standard deviation | Week 4 Mean ± standard deviation | p-value |
| ISI | 6.1 ± 2.6 | 6.0 ± 3.8 | 0.954 | 6.0 ± 2.3 | 4.4 ± 1.8 | 0.008 |

FIG. 3A is a graph showing results of measuring an amount of change in the Insomnia Severity Index (ISI) of the group taking the complex preparation of the mixed strain and the herbal medicinal material (Number Seven) and the placebo taking group according to an embodiment.

As shown in FIG. 3A, in the case of the group taking the complex preparation of the mixed strain and the herbal medicinal material (Number Seven) according to an embodiment, an ISI value showed a tendency to decrease throughout the administration period, and at week 4 of administration, it was confirmed that the amount of decrease in the ISI value was significantly greater than that of the placebo group. In addition, the decreasing tendency of the ISI value was maintained even after stopping the administration of the complex preparation of the mixed strain and the herbal medicinal material (Number Seven) (a wash-out period).

This means that the complex preparation of the mixed strain and the herbal medicinal material (Number Seven) according to an embodiment is effective in improving a sleep disorder.

### Experimental Example 4. Confirmation of effect of co-administration therapy of mixed strain and herbal medicinal material on defecation disorder improvement

To confirm a defecation disorder improvement effect upon co-administration of the mixed strain consisting of the *Lactobacillus* sp. strains and the herbal medicinal material, a Gut Quotient (GQ) scale evaluating a gut health of the participants recruited through the process of Experimental Example 2 was evaluated.

The Gut Quotient (GQ) scale is an index developed to evaluate gut health for Koreans, and is a questionnaire-based tool for evaluating a gut health state of a participant.

Specifically, the GQ is a validated questionnaire-based tool consisting of 17 items selected by clinicians and clinical experts based on items of Rome criteria, an Irritable Bowel Syndrome Severity Scoring System (IBS-SSS), and an Irritable Bowel Syndrome Quality of Life (IBS-QOL) questionnaire. The GQ measures gut, defecation, and defecation control discomforts and quantifies the same as a score, and a higher GQ score indicates better gut health.

Results of evaluating the Gut Quotient (GQ) scale of the group taking the complex preparation of the mixed strain and the herbal medicinal material (Number Seven) and the placebo taking group according to Experimental Example 2.2 are shown in Table 6 and FIG. 3B.

**[Table 6]**

| Measure | Placebo | | Complex preparation of mixed strain and herbal medicinal material (Number Seven) | | RM-ANOVA | | |
|---|---|---|---|---|---|---|---|
| | Mean ± standard deviation | | | | | | |
| | | | Mean ± standard deviation | | | | |
| | 0 wks | 4 wks | 0 wks | 4 wks | Group | Time | GroupX Time |
| Gut Quotient | 94.9 ± 5.8 | 92.0 ± 9.2 | 89.4 ± 12.7 | 93.8 ± 7.4 | 0.379 | 0.649 | 0.029* |

| | | | | | | | |
|---|---|---|---|---|---|---|---|
| (Mean ± SD,Two-way repeated measures ANOVA) | | | | | | | |

FIG. 3B is a graph showing results of evaluating an amount of change in the Gut Quotient (GQ) scale of the group taking the complex preparation of the mixed strain and the herbal medicinal material (Number Seven) and the placebo taking group according to an embodiment.

As shown in FIG. 3B, in the case of the group taking the complex preparation of the mixed strain and the herbal medicinal material (Number Seven) according to an embodiment, the GQ showed a tendency to increase during the administration period, and in particular, from week 2 of administration onward, it was confirmed that the amount of increase in the GQ of the group taking the complex preparation of the mixed strain and the herbal medicinal material (Number Seven) was significantly greater compared to the placebo group, in which the GQ decreased. In addition, the increasing tendency of the GQ was maintained even after stopping the administration of the complex preparation of the mixed strain and the herbal medicinal material (Number Seven) (a wash-out period).

This means that the complex preparation of the mixed strain and the herbal medicinal material (Number Seven) according to an embodiment is effective in improving a defecation disorder.

### Experimental Example 5. Confirmation of effect of co-administration therapy of mixed strain and herbal medicinal material on depression improvement

To confirm a depression improvement effect upon co-administration of the mixed strain consisting of the *Lactobacillus* sp. strains and the herbal medicinal material, a CES-D (Center for Epidemiological Studies-Depression Scale) of the participants recruited through the process of Experimental Example 2 was evaluated.

The CES-D (Center for Epidemiological Studies-Depression Scale) is a Center for Epidemiological Studies depression scale, and is a self-report questionnaire tool developed to evaluate a degree of depressive symptoms. The CES-D is widely used not only in a clinical setting but also in research, and is particularly used to identify a risk of developing depression or to evaluate a psychological state (depression level) in an epidemiological study.

Specifically, a revised Korean version of the Center for Epidemiological Studies depression scale was used, the CES-D consists of 20 items related to depression, and a score of each item ranges from 0 to 3. A higher total score means that depressive symptoms are more severe.

Results of evaluating the CES-D of the group taking the complex preparation of the mixed strain and the herbal medicinal material (Number Seven) and the placebo taking group according to Experimental Example 2.2 are shown in Table 7 and FIG. 3C.

**[Table 7]**

| Measure | Placebo | | | Complex preparation of mixed strain and herbal medicinal material (Number Seven) | | |
|---|---|---|---|---|---|---|
| | Week 0 Mean ± standard deviation | Week 4 Mean ± standard deviation | *P*-value | Week 0 Mean ± standard deviation | Week 4 Mean ± standard deviation | *P*-value |
| CESD | 3.7 ± 5.7 | 3.3 ± 5.3 | 0.740 | 6.1 ± 9.8 | 1.8 ± 2.3 | 0.053 |

| | | | | | | |
|---|---|---|---|---|---|---|
| (Mean ± SD, Paired t-test) | | | | | | |

FIG. 3C is a graph showing results of evaluating an amount of change in the CES-D of the group taking the complex preparation of the mixed strain and the herbal medicinal material (Number Seven) and the placebo taking group according to an embodiment.

As shown in FIG. 3C, in the case of the group taking the complex preparation of the mixed strain and the herbal medicinal material (Number Seven) according to an embodiment, the CES-D showed a tendency to decrease during the administration period, and in particular, from week 2 of administration of the complex preparation of the mixed strain and the herbal medicinal material (Number Seven) onward, it was confirmed that the amount of decrease in the CES-D was significantly large. In addition, the decreasing tendency of the CES-D was maintained even after stopping the administration of the complex preparation of the mixed strain and the herbal medicinal material (Number Seven) (a wash-out period).

This means that the complex preparation of the mixed strain and the herbal medicinal material (Number Seven) according to an embodiment is effective in improving depression.

### Experimental Example 6. Statistical analysis process according to co-administration of mixed strain and herbal medicinal material

Results of the co-administration of the mixed strain and the herbal medicinal material were analyzed using parametric multiple comparison procedures assuming normality of data. A two-way repeated measures analysis of variance (ANOVA) was performed, and accordingly, whether there was a change in an evaluation index according to medication progress and whether there was a difference between intervention factors were analyzed using a Bonferroni multiple comparison test as a post-hoc test. Statistics simply comparing only results before and after taking medication within an intervention factor, that is, between a state before taking medication and a state after taking medication for 4 weeks (Week 0 vs. Week 4), were analyzed by a paired t-test.

The above description of the present disclosure is for illustrative purposes, and those having ordinary skill in the art to which the present disclosure pertains will be able to understand that the present disclosure can be easily modified into other specific forms without changing the technical spirit or essential features of the present disclosure. Therefore, it should be understood that the embodiments described above are illustrative in all aspects and not restrictive.

### [Accession Number]

Name of Depositary Institution: Korea Research Institute of Bioscience and Biotechnology
Accession No.: KCTC14105BP
Date of Deposit: January 14, 2020

특허 출원을 위한 미생물 기탁의
국제적 승인에 대한 부다페스트 조약
국제적 양식
규칙 7.1 에 의거 발행된
원기탁에 대한 수탁증 원본
To. 지아이바이옴
지아이바이옴
167, 송파대로, 송파구, 서울특별시
대한민국
I. 미생물의 표시
기탁자에 의해 주어진 참조 표시:
Lactobacillus fermentum GB102 | 국제 기탁 기관에 의해 주어진 수탁번호:
KCTC 14105BP
II. 과학적 설명 및/또는 제안된 분류학적 명명
상기 I 에 표시된 미생물은 다음이 첨부된다:
[ ] 과학적 설명
[ ] 제안된 분류학적 명명
(적용되는 부분에 x 표시한다)
III. 수탁 및 수령
본 국제 기탁 기관은 상기 I 에 표시된 미생물을 수령하며, 그것은 2020. 01. 14 에
수탁되었다.
IV. 전환에 대한 요청의 수탁
I 로 특정된 미생물은 국제기탁기관에 수탁되었고, 동 미생물에 대한 원기탁을 부다페스트
조약에 의한 기탁으로의 전환 청구가 접수되었다.
V. 국제 기탁 기관
명칭: 한국 생물자원센터(KCTC)

주소: 한국생명공학연구원(KRIBB), 181, 입신길,
정읍시, 전라북도 56212 대한민국 | 국제 기탁 기관을 대표할 권한이 있는 자의
시명
담당자: 김성건
일자: 2020. 01. 14.

Name of Depositary Institution: Korea Research Institute of Bioscience and Biotechnology
Accession No.: KCTC14106BP
Date of Deposit: January 14, 2020

특허 출원을 위한 미생물 기탁의
국제적 승인에 대한 부다페스트 조약
국제적 양식
규칙 7.1 에 의거 발행된
원기탁에 대한 수탁증 원본
To. 지아이바이옴
지아이바이옴
167, 송파대로, 송파구, 서울특별시
대한민국

I. 미생물의 표시
기탁자에 의해 주어진 참조 표시:
Lactobacillus fermentum GB103 | 국제 기탁 기관에 의해 주어진 수탁번호:
KCTC 14106BP
II. 과학적 설명 및/또는 제안된 분류학적 명명
상기 I 에 표시된 미생물은 다음이 첨부된다:
[ ] 과학적 설명
[ ] 제안된 분류학적 명명
(적용되는 부분에 x 표시한다)
III. 수탁 및 수령
본 국제 기탁 기관은 상기 I 에 표시된 미생물을 수령하며, 그것은 2020. 01. 14 에
수탁되었다.
IV. 전환에 대한 요청의 수탁
I 로 특정된 미생물은 국제기탁기관에 수탁되었고, 동 미생물에 대한 원기탁을 부다페스트
조약에 의한 기탁으로의 전환 청구가 접수되었다.
V. 국제 기탁 기관
명칭: 한국 생물자원센터(KCTC)

주소: 한국생명공학연구원(KRIBB), 181, 입신길,
정읍시, 전라북도 56212 대한민국 | 국제 기탁 기관을 대표할 권한이 있는 자의
시명
담당자: 김성건
일자: 2020. 01. 14.

Name of Depositary Institution: Korea Research Institute of Bioscience and Biotechnology
Accession No.: KCTC14107BP
Date of Deposit: January 14, 2020

특허 출원을 위한 미생물 기탁의
국제적 승인에 대한 부다페스트 조약
국제적 양식
규칙 7.1 에 의거 발행된
원기탁에 대한 수탁증 원본
To. 지아이바이옴
지아이바이옴
167, 송파대로, 송파구, 서울특별시
대한민국
I. 미생물의 표시
기탁자에 의해 주어진 참조 표시:
Lactobacillus plantarum GB104 | 국제 기탁 기관에 의해 주어진 수탁번호:
KCTC 14107BP
II. 과학적 설명 및/또는 제안된 분류학적 명명
상기 I 에 표시된 미생물은 다음이 첨부된다:
[ ] 과학적 설명
[ ] 제안된 분류학적 명명
(적용되는 부분에 x 표시한다)
III. 수탁 및 수령
본 국제 기탁 기관은 상기 I 에 표시된 미생물을 수령하며, 그것은 2020. 01. 14 에
수탁되었다.
IV. 전환에 대한 요청의 수탁
I 로 특정된 미생물은 국제기탁기관에 수탁되었고, 동 미생물에 대한 원기탁을 부다페스트
조약에 의한 기탁으로의 전환 청구가 접수되었다.
V. 국제 기탁 기관
명칭: 한국 생물자원센터(KCTC)

주소: 한국생명공학연구원(KRIBB), 181, 입신길,
정읍시, 전라북도 56212 대한민국 | 국제 기탁 기관을 대표할 권한이 있는 자의
시명
담당자: 김성건
일자: 2020. 01. 14.

## Claims

1. A composition for preventing or alleviating a sleep disorder, comprising, as active ingredients, a first active material comprising a *Lactobacillus* sp. strain or a culture thereof and a second active material comprising an herbal medicinal material; or
comprising the first active material as an active ingredient, wherein the second active material is administered in combination.

2. The composition for preventing or alleviating a sleep disorder according to claim 1, further comprising a third active material comprising inulin or a vitamin; or
wherein a third active material is further administered in combination.

3. The composition for preventing or alleviating a sleep disorder according to claim 1, wherein the first active material is *Lactobacillus fermentum* and *Lactobacillus plantarum*.

4. The composition for preventing or alleviating a sleep disorder according to claim 3, wherein the first active material further comprises a *Bifidobacterium animalis* subsp. *lactis* strain and a *Lactobacillus acidophilus* strain.

5. The composition for preventing or alleviating a sleep disorder according to claim 1, wherein the second active material is at least one selected from the group consisting of Chinese yam, adlay, red ginseng, Reishi mushroom, and a combination thereof.

6. The composition for preventing or alleviating a sleep disorder according to claim 1 or 2, wherein the first active material, the second active material, and the third active material are administered in combination simultaneously, sequentially, or in a reverse order.

7. The composition for preventing or alleviating a sleep disorder according to claim 1 or 2, comprising a first oral preparation comprising the first active material, a second oral preparation comprising the second active material, and a third oral preparation comprising the third active material, wherein the first, second, and third oral preparations are orally administered.

8. The composition according to claim 7, wherein the oral preparation is in a form of tablets, pills, capsules, lozenges, granules, powders, suspensions, sachets, or syrups.

9. The composition according to claim 1, wherein the strain is lyophilized and included in an amount of 10³ to 10¹⁶ CFU/g.

10. A health functional food for preventing or alleviating a sleep disorder, comprising, as active ingredients, a first active material comprising a *Lactobacillus* sp. strain or a culture thereof and a second active material comprising an herbal medicinal material; or
comprising the first active material as an active ingredient, wherein the second active material is administered in combination.

11. A method of preventing or alleviating a sleep disorder, comprising: administering a first active material comprising a *Lactobacillus* sp. strain or a culture thereof to a subject in need thereof; and
administering a second active material comprising an herbal medicinal material to the subject in need thereof.

12. Use of the composition of claim 1 for the manufacture of a medicament for preventing or alleviating a sleep disorder.

13. Use of the composition of claim 1 for preventing or alleviating a sleep disorder.
